**Europäisches Patentamt**

**European Patent Office**

**Office européen des brevets**

(19)

(11) Publication number : **0 327 299 B1**

(12) # EUROPEAN PATENT SPECIFICATION

(45) Date of publication of patent specification :
16.09.92 Bulletin 92/38

(51) Int. Cl.⁵ : **A61M 25/02**

(21) Application number : **89300905.0**

(22) Date of filing : **31.01.89**

(54) Catheter securing device.

(30) Priority : **04.02.88 GB 8802480**

(43) Date of publication of application :
**09.08.89 Bulletin 89/32**

(45) Publication of the grant of the patent :
**16.09.92 Bulletin 92/38**

(84) Designated Contracting States :
**AT BE DE ES FR GB IT NL SE**

(56) References cited :
**EP-A- 0 037 198**
**EP-A- 0 274 418**
**US-A- 4 519 793**
**US-A- 4 579 120**

(73) Proprietor : **BARD LIMITED**
**Pennywell Industrial Estate**
**Sunderland SR4 9EW (GB)**

(72) Inventor : **Brazier, Gary Brian**
**10 Main Road Great Oakley**
**Harwich Essex CO12 5AZ (GB)**

(74) Representative : **Jackson, Peter et al**
**HYDE, HEIDE & O'DONNELL 10-12 Priests**
**Bridge**
**London SW15 5JE (GB)**

## Description

This invention relates to a device for holding a medical catheter in position at the point where it enters a patient's body.

The term catheter is employed in this description to refer to all types of hollow tubular units employed for removal of bodily fluids or for introduction of fluids into the body. At the point on the patient's skin surface where a catheter passes into the body there is a tendency for the catheter to move relative to the surrounding tissue. This not only causes discomfort for the patient but can produce such problems as skin maceration, skin irritation, kinks in the catheter or prevention of scab formation.

Various means have been adopted to secure the catheter relative to the body at this entry point. These include directly securing the catheter to the adjacent skin by sutures or adhesive tape but these can create dirt traps which lead to irritation and infection. A proposal that overcomes some of the problems is to place a resilient button on the skin surface at the point of entry, securing the catheter firmly to the button and securing the rim of the button to the skin surface, thereby distancing the skin attachment region from the skin entry point.

Previous proposals for such buttons have however given rise to the difficulty that in order to provide adequate securing of the catheter to the button the securing means have been cumbersome and obtrusive. As a result they have tended to snag the patient's clothing or bed linen, thus putting the button/skin securing means under strain, again causing discomfort and at worst dislodging the catheter.

US patent specification 4759120 discloses a molded retaining member for anchoring a flexible percutaneous lead which exits the body of a patient, most particularly an electrically conductive lead such as used for a heart pacemaker. The retaining member has a flexible disc portion and, perpendicular thereto, a stem and a bulbous head portion. The patent is particularly concerned with avoiding the use of sutures and achieves this by employing an adhesive layer on the exterior surface of the disc portion to adhere to the patient's body. A passage extends longitudinally through the adhesive, the disc and the head portion for snugly receiving the lead. The lead thus emerges from the retaining member in a direction perpendicular to the disc.

The present invention provides an unobtrusive, smooth-profile catheter button which can be comfortably worn under normal clothing.

According to the invention there is provided a catheter securing device which comprises a generally cylindrical button formed of resilient material, characterised in that the button includes a guide channel (21) for the catheter, which channel passes through the button from its curved cylindrical surface to its base, and further includes a slot (25) with substantially parallel opposing faces which extends from the top of the button to the guide channel (21) and a band (17) to be secured around the cylindrical surface above the guide channel (21) so as to draw together the opposing faces of the slot (27) and to hold the catheter firmly in the guide channel (21).

The resilient material is preferably a medically approved elastomer, for example silicone rubber, soft polyvinyl chloride or polyurethane. The elastomeric properties impart several advantages to the button. Thus they assist the button to grip the catheter surface, thereby resisting relative movement of the button and catheter, they permit a button of given dimensions to accommodate a range of different diameter catheters, they allow the button to conform to the contours of the body, including changes due to body movement, and thus make it comfortable to wear. They also give the button a pleasant softness in touch, further enhancing the comfort to the patient.

The button is preferably in the shape of a cylinder, with a height less than one half of its diameter. For convenience of description herein the face of the button to contact the skin is referred to as the base and the face remote from the skin is referred to as the top, although these will not necessarily be accurate descriptions of the relative positions of those faces when the button is in position on the patient. This description also implies a circular shape for the button but it is to be understood that other shapes, while not preferred, are acceptable.

The catheter guide channel through the button preferably follows a gentle curve from the base to the side (cylindrical) face, so as to avoid kinks in the catheter and to allow the catheter to emerge from the side face substantially parallel to the skin surface.

The base portion of the button preferably incorporates a circumferential flange so as to increase the area of contact between the skin surface and the button. The flange diameter is preferably at least twice that of the central cylindrical portion.

Attachment of the button to the skin can be effected by sutures, by incorporating a self-adhesive coating on the base of the button or by the use of adhesive tape. To facilitate location and insertion of sutures the flange can be advantageously be provided with several small holes around its periphery. In one preferred version of this embodiment the holes do not fully extend through the flange and thereby leave a membrane in place in each of the holes. The membrane assists in preventing build up of dirt and infection in the holes, including those through which a suture is passed.

At the point where the catheter enters the base of the button there is preferably an indentation in the button to leave space for scab formation. The indentation preferably has a spherical surface, with the catheter channel leading from the uppermost part of this surface.

One or more circular ribs can be provided around the periphery of the flange. These add rigidity which resists distortion of the periphery as the securing band is tightened and thereby facilitate good contact around the whole of the periphery with the adjacent skin surface. They also reduce any prospect of a suture passed through the peripheral holes from tearing through the edge of the flange.

The location of the slot in the button is preferably such that it forms a diameter of the upper part of the button. In those versions of button including a flange the slot preferably also extends from one side of the central cylindrical portion of the button to the edge of the flange so as to permit the button to be put into position or removed without removing the catheter. The width of the slot is preferably less than the diameter of the catheter guide channel so as to provide a shoulder where the guide channel meets the slot and thereby help in keeping the catheter in place in the channel.

The catheter guide channel can be sized to allow a measure of choice in the catheter diameter, typically to permit a range of two or three standard catheter diameters. This is achieved by making the catheter guide channel diameter slightly less than the diameter of the largest desired catheter, using the resilience of the button material to permit insertion of the largest catheter and using the ability to reduce the channel diameter, by drawing together the faces of the slot, to hold the smaller diameter catheters.

The securing band to be placed around the cylindrical button surface can be for example an adhesive strip or a tie-up thread but is preferably a plastic buckle band. Most preferably the buckle is of a non-slip type in which grooves across the band engage a retaining bar in the buckle such that once inserted the band cannot be removed from the buckle. The cylindrical portion of the button preferably includes a circumferential groove to receive the band and preferably also a recess in the cylindrical surface to receive the buckle. The recess keeps the buckle out of contact with the patient's clothing and also makes for easy insertion of scissors to cut the band when the button is to be removed. A retaining bar is preferably located on the cylindrical button surface so as to hold the securing band on the button prior to use.

Apart from the securing band all other portions of the button are preferably formed in one piece. All edges of the button are preferably given a smooth shape and finish to minimise the possibility of snagging the patient's clothing.

The buttons according to the invention offer a number of advantages in easy attachment and removal, prevention of dirt build-up, avoidance of being dislodged and, above all, in comfort for the patient.

The invention is described below by way of example with reference to the accompanying drawings, in which;

Figure 1 is a plan view of a button according to the invention.

Figure 2 is a cross section of the button of figure 1 taken along the line X - X.

Figure 3 is an elevation of the button viewed from position Y on figure 1.

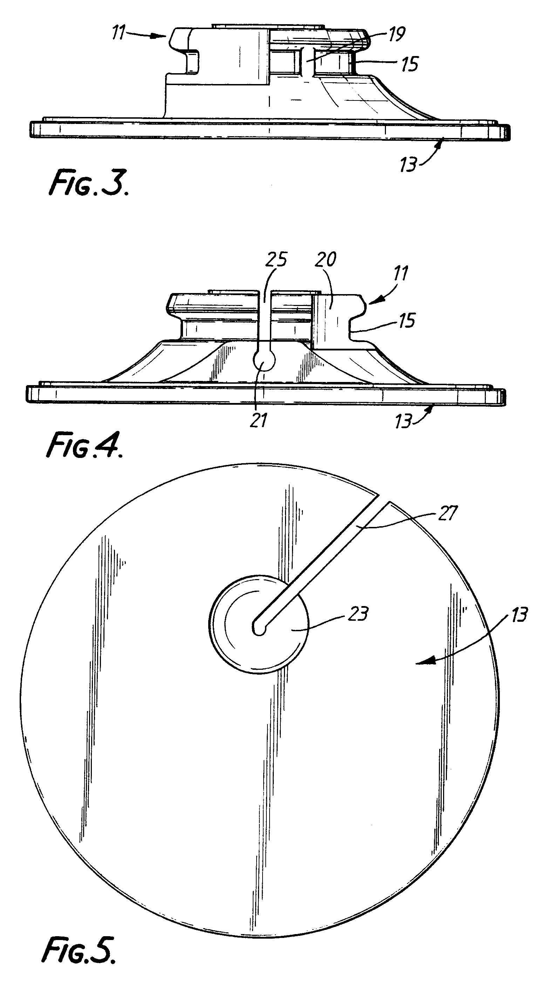

Figure 4 is an elevation of the button viewed from position Z on figure 1.

Figure 5 is a view of the underside of the button.

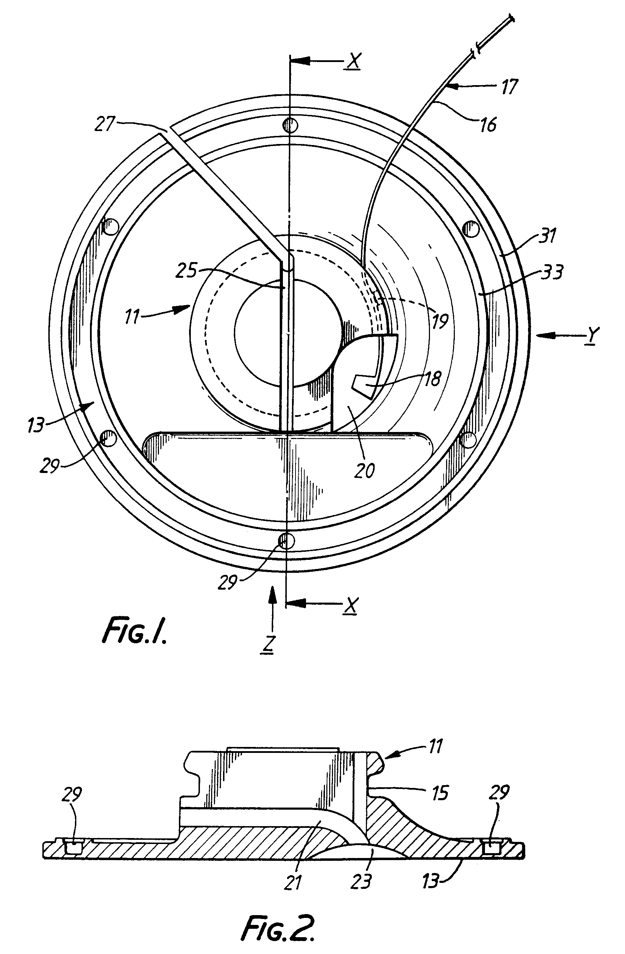

The button shown in figures has a central cylindrical portion 11 and a base flange 13. A groove 15 is provided around the cylinder 11 to receive a securing band 17 (shown in figure 1 in position prior to use). The band 17 is of the type with a notched strap 16 and retaining buckle 18. A retaining bar 19 is provided across the groove 15 to hold the band 17 ready for use and a recess 20 is provided in the cylindrical portion 11 to receive the buckle 18 when the band 17 is secured.

A catheter channel 21 leads from the side of the portion 11, to the base of the button, opening into the centre of an indentation 23. Above the channel 21 a slot 25 extends to the top of the button. A further slot 27 leads from the centre of the indentation 23 to the edge of the flange 13.

Suture securing holes 29 are provided around the periphery of the flange 13 but do not extend fully through it (see figure 2). The flange 13 also includes circular ribs 31 and 33 and is shaped to curve smoothly upwards from the inner rib 33 to the side of the portion 11 at the lower edge of groove 15.

In use, with a catheter already in place in the patient, the button is placed over the catheter by means of slots 27 and 25 and the catheter is located in the channel 21. The securing band 17 is then put in position in groove 15 and the buckle 18 is located in recess 20. The tightness of the band 17 is increased to the point at which the catheter is firmly held in the channel 21. If desired the button can be secured to the skin by sutures inserted through holes 29.

Removal of the button is the reverse of the above procedure, following severing of the securing band 17 and of any sutures.

## Claims

1. A catheter securing device which comprises a generally cylindrical button formed of resilient material, characterised in that the button includes a guide channel (21) for the catheter, which channel passes through the button from its curved cylindrical surface to its base, and further includes a slot (25) with substantially parallel opposing faces which extends from the top of the button to the guide channel (21) and a band (17) to be secured around the cylindrical surface above the guide channel (21) so as to draw together the opposing faces of the slot (27) and to hold the catheter firmly in the guide channel (21).

2. A catheter securing device as claimed in claim 1, in which the button incorporates a circumferential flange (13).

3. A catheter securing device as claimed in claim 2, in which the flange is provided with several small holes (29) around its periphery.

4. A catheter securing device as claimed in claim 3, in which the holes (29) do not fully extend through the flange (13).

5. A catheter securing device as claimed in any one of claims 2 to 4, in which one or more circular ribs (31, 33) are provided around the periphery of the flange.

6. A catheter securing device as claimed in any preceding claim, in which the slot (25) forms a diameter of the upper part (11) of the button.

7. A catheter securing device as claimed in any preceding claim, in which the button includes a circumferential groove (15) to receive the band (17).

8. A catheter securing device as claimed in any preceding claim, in which the securing band (17) is a plastic buckle band.

9. A catheter securing device as claimed in claim 8, in which the button includes a recess (20) to receive the buckle (18).

10. A catheter securing device as claimed in any any preceding claim, in which a bar (19) to retain the securing band (17) is located on the cylindrical surface of the button.

**Patentansprüche**

1. Befestigungsvorrichtung für Katheter mit einem im wesentlichen zylindrischen Knopf aus elastischem Material, **dadurch gekennzeichnet,** daß der Knopf einen Führungskanal (21) für den Katheter aufweist, welcher Kanal den Knopf von seiner gekrümmten zylindrischen Oberfläche bis zu seiner Basis durchsetzt und weiterhin einen Schlitz (25) mit im wesentlichen parallel zueinander verlaufenden gegenüberliegenden Flächen aufweist, der sich von der Oberseite des Knopfs bis zu dem Führungskanal (21) erstreckt sowie mit einem Band (17), das um die zylindrische Oberfläche oberhalb des Führungskanals (21) herum befestigt ist zum Zusammenziehen der einander gegenüberstehenden Flächen des Schlitzes (27) derart, daß der Katheter in dem Führungskanal (21) festgehalten ist.

2. Katheterbefestigungsvorrichtung nach Anspruch 1, bei welcher der Knopf einen umlaufenden Randflansch (13) aufweist.

3. Katheterbefestigungsvorrichtung nach Anspruch 2, bei welcher der Flansch entlang seiner Peripherie mit mehreren kleinen Löchern (29) versehen ist.

4. Katheterbefestigungsvorrichtung nach Anspruch 3, bei welcher die Löcher (29) den Flansch (13) nicht voll durchsetzen.

5. Katheterbefestigungsvorrichtung nach einem der Ansprüche 2 bis 4, bei welcher eine oder mehrere ringförmige Rippen (31,33) um die Peripherie des Flansches vorhanden sind.

6. Katheterbefestigungsvorrichtung nach einem der vorstehenden Ansprüche, bei der der Schlitz (25) in einem Durchmesserbereich des oberen Teils (11) des Knopfes ausgebildet ist.

7. Katheterbefestigungsvorrichtung nach einem der vorstehenden Ansprüche, bei welcher der Knopf zur Aufnahme des Bands (17) mit einer umlaufenden Nut (15) versehen ist.

8. Katheterbefestigungsvorrichtung nach einem der vorstehenden Ansprüche, bei welcher das Befestigungsband (17) ein riemenartiges Kunststoffband ist.

9. Katheterbefestigungsvorrichtung nach Anspruch 8, bei welcher der Knopf eine Aussparung (20) zur Aufnahme von einer dem Band zugeordneten Schnalle (18), Spange oder dergleichen versehen ist.

10. Katheterbefestigungsvorrichtung nach einem der vorstehenden Ansprüche, bei der an der zylindrischen Umfangsfläche des Knopfs ein Steg (19) zum Halten des Befestigungsbands (17) ausgebildet ist.

**Revendications**

1. Dispositif de fixation d'un cathéter, comportant un bouton de forme générale cylindrique en un matériau élastique, caractérisé en ce que le bouton comporte un canal de guidage (21) pour le cathéter, qui passe à travers le bouton depuis sa surface cylindrique jusqu'à sa base et qui comprend en outre une fente (25) dont les faces opposées sont sensiblement parallèles et qui s'étend depuis le sommet du bouton jusqu'au canal de guidage (21) et une bande (18) destinée à être fixée autour de la surface cylindrique au-dessus du canal de guidage (21) de manière à rapprocher les faces opposées de la fente (27) et à maintenir fermement le cathéter dans le canal de guidage (21).

2. Dispositif de fixation de cathéter selon la revendication 1, dans lequel le bouton comporte une bride circonférentielle (13).

3. Dispositif de fixation de cathéter selon la revendication 2, dans lequel plusieurs petits orifices (29) sont ménagés dans la bride le long de sa périphérie.

4. Dispositif de fixation de cathéter selon la revendication 3, dans lequel les orifices (29) ne traversent pas l'épaisseur de la bride (13).

5. Dispositif de fixation de cathéter selon l'une quelconque des revendications 2 à 4, dans lequel une ou plusieurs nervures circulaires (31, 33) sont prévues autour de la périphérie de la bride.

6. Dispositif de fixation de cathéter selon l'une quelconque des revendications précédentes, dans lequel la fente (25) est formée le long d'un diamètre de la partie supérieure (11) du bouton.

7. Dispositif de fixation de cathéter selon l'une quelconque des revendications précédentes, dans lequel le bouton comporte une gorge circonférentielle (15) pour recevoir la bande (17).

8. Dispositif de fixation de cathéter selon l'une quelconque des revendications précédentes, dans lequel la bande de fixation (17) est une sangle à boucle en plastique.

9. Dispositif de fixation de cathéter selon la revendication 8, dans lequel est ménagé un évidement (20) pour recevoir la boucle (18).

10. Dispositif de fixation de cathéter selon l'une quelconque des revendications précédentes, dans lequel une tige (19) pour retenir la bande de fixation (17) est logée sur la surface cylindrique du bouton.

*Fig.I.*

*Fig.2.*

FIG.3.

FIG.4.

FIG.5.